# EUROPEAN PATENT APPLICATION

(11) **EP 4 621 393 A1**
(43) Date of publication of application: **24.09.2025**
(21) Application number: 24164140.6
(22) Date of filing: 18.03.2024
(51) Int. Cl.: G01N 22/04, G01N 33/15

(54) **METHOD AND SYSTEM FOR DETERMINING THE MOISTURE CONTENT OF A PHARMACEUTICAL PRODUCT, AND METHOD AND SYSTEM FOR PRODUCING A PHARMACEUTICAL PRODUCT**

(71) Applicant: LTS Lohmann Therapie-Systeme AG, 56626 Andernach (DE)
(72) Inventor: Scherr, Sebastian, 56335 Neuhäusel (DE); Kuch, Christopher, 56410 Montabaur (DE); Heidary Dastjerdi, Maral, 53424 Remagen (DE)
(74) Representative: dompatent von Kreisler Selting Werner - Partnerschaft von Patent- und Rechtsanwälten mbB

(57) **Abstract**

The invention relates to a method for determining the moisture content of a pharmaceutical product comprising the steps of emitting a signal into the pharmaceutical product; and measuring a signal response based on an influence of the pharmaceutical product on the signal. Further, the invention relates to a method and system for producing a pharmaceutical product as well as to a system (10) for determining the moisture content of a pharmaceutical product.

## Description

The present invention relates to a method and a system for determining the moisture content of a pharmaceutical product as well as to a method and a system for producing a pharmaceutical product.

Drying is a key factor in manufacturing pharmaceutical products, particularly pharmaceutical patches such as microneedle patches, oral films, or transdermal patches.

On the one hand, drying has a direct influence on the quality and thus the performance of the pharmaceutical products. For example, drying influences the mechanical stability, durability, as well as the dissolving behavior.

On the other hand, drying, in particular the drying time, accounts for a large proportion of the production costs, e.g. as it directly influences the process time.

Thus, it is an object of the present invention to provide a method and a system to optimize the production of pharmaceutical products, particularly of pharmaceutical patches.

The object is solved by a method for determining the moisture content of a pharmaceutical product according to claim 1, a method for producing a pharmaceutical product according to claim 8, a system for determining the moisture content of a pharmaceutical product according to claim 14, and a system for producing a pharmaceutical product according to claim 15.

The method for determining the moisture content of a pharmaceutical product, particularly corresponds to a method for determining the moisture content of a pharmaceutical patch. It is preferred that the method is a method which is integrated, particularly inline, into the production of the pharmaceutical product, whereby the method is particularly carried out during the production run. The method is preferably a non-destructive method. Thus, preferably, the pharmaceutical product is not destroyed and/or altered and is particularly in a condition to be applicable after carrying out the method. The method comprises emitting a signal into the pharmaceutical product. Particularly the signal corresponds to electromagnetic waves and/or microwaves. It is preferred that the method comprises generating an electromagnetic field, whereby the signal is emitted. The method further comprises the step of measuring a signal response based on an influence of the pharmaceutical product on the emitted signal. Thus, it is preferred that a response of the emitted signal is measured. Preferably, the measuring step comprises to measure an alteration of the emitted signal due to the presence of the pharmaceutical product, particularly due to the dielectric properties of the pharmaceutical product.

Preferably, the measuring step comprises to measure a shift of a resonance frequency, particularly caused by the presence of the pharmaceutical product. It is preferred that a resonance frequency is produced and a deviation of the resonance frequency due to the presence of the pharmaceutical product is measured.

Preferably, the method comprises the step of evaluating, particularly determining, the moisture content of the pharmaceutical product based on the measured signal response. For example, the method comprises comparing the measured response, preferably the shift of the resonance frequency, with a set of data to determine a moisture value corresponding to the measured response.

Preferably, at least one resonator, is used to carry out the method, particularly to determine the moisture content. It is preferred that the resonator is used to emit the signal and/or receive, particularly measure, the response. The resonator is preferably adapted to produce an electric, particularly an electromagnetic, field. The resonator is, for example, a split-cavity resonator and/or a stray field resonator. It is preferred that a plurality, preferably a plurality of different, resonators is used to carry out the method.

Preferably the resonator comprises an RC-resonator and/or an LC-circuit.

Preferably, the pharmaceutical product is arranged in a measuring zone of the resonator by means of a carrier holding the pharmaceutical product.

Preferably, the method comprises the steps of measuring a response, particularly a shift of the resonance frequency, of the signal of at least one sample having a known moisture content. Based on the measured response a calibration data is generated, preferably stored, for example as the set of data. Particularly this measuring and data generation step is repeated for a plurality of samples having a different know moisture content, particular to generate a set of calibration data, for example a calibration curve. The at least one sample preferably corresponds to a pharmaceutical product. Preferably, the calibration data is stored in a control, particularly a control device connected to the resonator.

Preferably, the method comprises the further step of performing a Karl-Fischer analysis to determine the moisture content of the at least one sample. This step is particularly performed before measuring the response of the sample. Preferably this step is carried out to determine the moisture of the sample, leading to the sample having a known moisture content.

The method for manufacturing a pharmaceutical product particularly corresponds to a method for manufacturing a pharmaceutical patch. The method comprises fabricating, particularly casting and/or assembling, the pharmaceutical product. Particularly, the product is molded. Further the method comprises carrying out the method for determining the moisture content of a pharmaceutical product, preferably to determine the moisture content of the pharmaceutical product. The method for determining the moisture content is preferably carried out after a predefined time after fabricating the product, particularly during a drying step of the fabricated product, for example after 1 to 24 hours, preferably after 2 to 12 hours, more preferably after 4 to 9 hours.

Preferably, the method comprises the step of determining at least one drying parameter based on the determined moisture content. The drying parameter particularly comprises a drying time and/or a drying temperature, a drying air flow. The drying time preferably corresponds to a time amount required to reach a desired moisture level of the product.

Preferably, the method comprises the step of drying the pharmaceutical product based on the determined moisture content. Particularly, the drying is carried out based on the one determined drying parameter. The drying step particularly comprise a resting time.

Preferably, drying is terminated if a predefined moisture content is determined by the step of carrying out the method for determining the moisture content of a pharmaceutical product. Thus, particularly if the predefined moisture content is determined for the product, drying is completed and preferably the product is processed further, for example demolded and/or packaged. Preferably, the method comprises the step of demolding and/or of packaging the pharmaceutical product after drying, particularly after drying is terminated based on the determined moisture content of the pharmaceutical product.

Preferably, the step of determining the moisture content of the pharmaceutical product is carried out at various positions in the fabrication process and/or at various time points during the fabrication process. For example, the step of determining the moisture content may be carried out on a pre-product before fabrication and/or on an intermediate product during fabrication and/or on the fabricated product, preferably during drying of the fabricated product. Particularly, the step of determining the moisture content may be carried out continuously, for example continuously during drying, preferably until a predefined moisture content is reached.

Preferably, the method is a method of manufacturing a, preferably dissolvable, microneedle array. The fabricating step of manufacturing the microneedle array particularly comprises casting a microneedle tip, preferably a plurality of microneedle tips, and/or the fabricating step of manufacturing the microneedle array comprises casting a backing layer connected to the microneedle tip. Casting of the microneedle tip and/or the backing layer is preferably carried out in a microneedle mold. It is thus preferred that before casting the microneedle tip a mold for casting the microneedle array is provided. The mold particularly comprises, preferably consists of, non-metallic material and/or polymer and/or ceramic. Preferably, materials which have a magnetic but no electrical conductivity are used. For example, materials made of silicone, ceramics, and plastics can be used. One or more intermediate layers may be casted between the microneedle tip and the backing layer.

Preferably, the step of determining the moisture content, particularly the steps of emitting the signal and measuring the response, is carried out before casting the microneedle tip, and/or between casting the microneedle tip and casting the backing layer, and/or after casting the backing layer. For example, the signal may be emitted into the empty mold to measure a corresponding response. Thus, it is preferably possible to determine manufacturing tolerances of the mold. Alternatively, or in addition, the signal may be emitted into the fabricated product and/or an intermediate product during fabricating.

The system for determining the moisture content of a pharmaceutical product particularly corresponds to a system for determining the moisture content of a pharmaceutical patch. The system comprises at least one resonator and a carrier for the pharmaceutical product. The carrier is adapted to arrange the pharmaceutical product in the measuring zone of the resonator. The resonator is, for example, a split-cavity resonator and/or a stray field resonator. It is preferred that a plurality, preferably a plurality of different, resonators is used to carry out the method. Preferably the resonator comprises an RC-resonator and/or an LC-circuit.

The system for manufacturing a pharmaceutical product, particularly a pharmaceutical patch, comprises the system for determining the moisture content of a pharmaceutical product, and a manufacturing device, particularly a casting device, for the production of the pharmaceutical product. In an alternative preferred adaptation of the system for manufacturing a pharmaceutical product according to the invention it is not necessary that the system for manufacturing a pharmaceutical product comprises a carrier. The system preferably corresponds to a production line, particularly a conveyor belt production line. The casting device particularly comprises a drop dispenser, preferably to produce microneedles, more preferably microneedle tips; and/or an extruder, particularly a screw extruder, preferably to produce a microneedle patch, more preferably the backing layer of a microneedle patch. It is preferred that the system comprises a drying station, particularly arranged after the casting device, preferably in the production line. Preferably, the system comprises a demolding device for demolding the pharmaceutical product and/or a packaging station, particularly a packaging device, for packaging the pharmaceutical product. It is preferred that the resonator is arranged in front of the casting device and/or after the casting device and/or within the drying station and/or after the drying station and/or in front of the demolding device and/or in front of the packaging station, preferably in the production line.

Preferably, the pharmaceutical patch to be analyzed by the method and/or the system for determining the moisture content of a pharmaceutical product and/or to be manufactured by the system and/or the method for manufacturing a pharmaceutical product corresponds to a microneedle patch and/or an oral film and/or a transdermal patch, also called transdermal therapeutic system. The microneedle patch particularly corresponds to microneedle patch having dissolving microneedles, hydrogel microneedles and/or coated microneedles, e.g. coated, ceramics microneedles. The microneedles preferably comprise, more preferably consist of, polymer and/or ceramics, particularly in combination with an active ingredient.

Preferably, the carrier used in the method and/or the system for determining the moisture content of a pharmaceutical product and/or in the system and/or the method for manufacturing a pharmaceutical product is adapted to arrange the pharmaceutical product in a fixed position if the carrier is arranged, particularly inserted, in a carrier receiving portion of the resonator. Particularly, the carrier is adapted to arrange the product in a single and/or unambiguous fixed position. The carrier may comprise a fixation, such as an indentation, for arranging the pharmaceutical product in a fixed position. The carrier comprises or consists of a carrier plate and/or a conveyor belt and/or a film web. The carrier comprises, more preferably consist of, polymer and/or ceramics. It is preferred that the carrier is non-metallic.

It is preferred that one or more objects according to the invention, i.e. the methods and/or the systems, comprise one or more features of the other objects according to the invention.

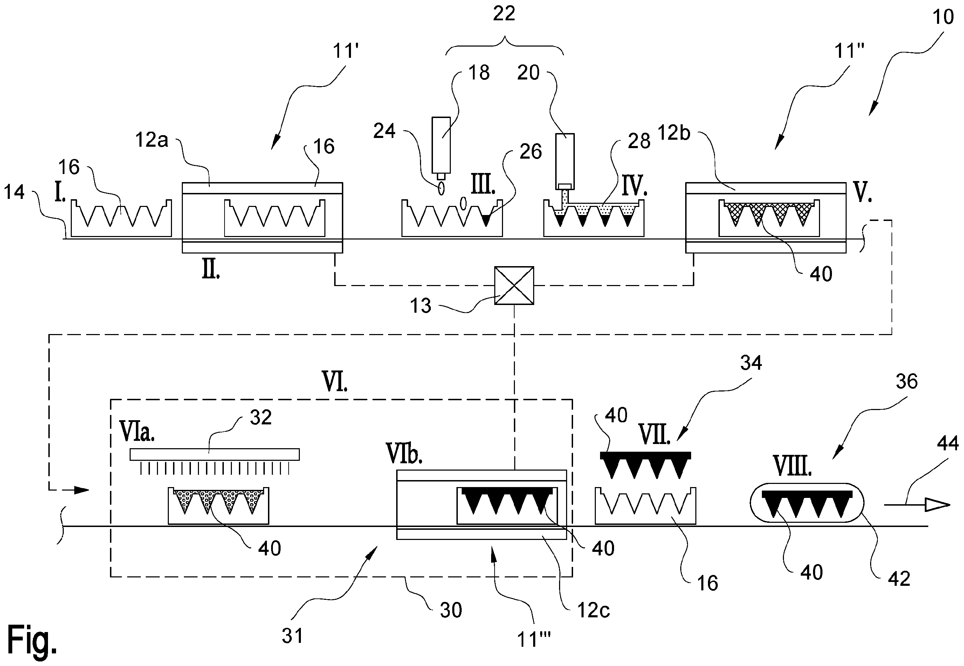

In the following the present invention is described in more detail with reference to the accompanying drawing.

The figure shows: a schematic drawing of a preferred embodiment of a system 10 for manufacturing a pharmaceutical product including preferred embodiments of a system 11', 11", 11‴ for determining the moisture content of the pharmaceutical product.

The figure also shows a preferred embodiment method for producing a pharmaceutical product including preferred embodiments of a method for determining the moisture content of a pharmaceutical product.

The system 10 shown in the figure is a system for manufacturing a microneedle array patch 40.

The system 10 corresponds to a production line comprising a carrier 14 in form of a conveyor belt for holding the pharmaceutical product to be manufactured, whereby the process flow direction is indicated by arrow 44.

A microarray mold 16 is provided on the conveyor belt 14, which preferably corresponds to a first method step I. Mold 16 comprises cavities for the microneedle array patch 40 to be casted.

The microarray mold 16 is inserted into a resonator 12a. Preferably the resonator 12a is adapted to measure a shift of a resonance frequency caused by the presence of the mold 16, for example to determine manufacturing tolerances of different molds 16. This process preferably corresponds to a second method step II.

Next, mold 16 is filled with drops 24 of a formulation, preferably containing an active ingredient, by a drop dispenser 18 to cast the tips 26 of the microneedle array patch 40. This process preferably corresponds to a third method step III.

An extruder 20, preferably a screw extruder, fills the remaining cavities with a, preferably non-active ingredient containing, formulation to produce a backing layer 28 for the microneedle array patch 40. This process preferably corresponds to a fourth method step IV.

Extruder 20 and drop dispenser 18 together correspond to a casting device 22.

Next, the filled mold 16 is inserted into a resonator 12b. Resonator 12b is preferably adapted to determine a moisture content of the microarray 40 after molding. The microarray 40 is shown in a state after molding and/or before drying. It is preferred that at least one drying parameter is determined based on moisture content determined by resonator 12b. The drying parameter particularly comprises a drying time, a drying temperature, and/or a drying air flow. This process preferably corresponds to a fifth method step V.

After that the molded microarray 40 is dried in drying station 30.

Drying station 30 comprises a heating device 32 to dry the microarray 40, preferably corresponds sub step VIa. of a sixth method step VI. Here, the microarray 40 is shown in a state during drying.

Further, drying station 30 comprises a resting station 31, whereby the microarray 40 is dried over a, preferably predefined, drying time. This drying process preferably corresponds a sub step VIb. of the sixth method step VI. In the drying station 30, preferably resting station 31, a resonator 12c determines the moisture content of microarray 40. It is preferred that the moisture content of microarray 40 is continuously determined by resonator 12c. Particularly, if a predefined moisture content of microarray 40 is reached, drying is terminated. Here, the microarray 40 is shown in a dried state.

After terminating drying, the microarray 40 is demolded from mold 16, preferably by means of a demolding device 34 (not shown). This process preferably corresponds to a seventh method step VII.

The demolded microarray 40 is then packaged in a packaging 42, particularly by means of a packaging device 36 (not shown). This process preferably corresponds to an eighth method step VIII.

Resonators 12a, 12b, 12c are preferably adapted to create an electromagnetic field and measure a shift of a resonance frequency caused by the presence of the mold 16 and/or the microneedle array patch 40. Further, it is preferred that resonators 12a, 12b, 12c are connected, preferably in data transmission, to a control device 13. The control device 13 preferably stores calibration data to assign the measured shift of the resonance frequency to corresponding moisture content.

## Claims

1. Method for determining the moisture content of a pharmaceutical product, particularly of a pharmaceutical patch (40), the method comprising the steps of:
- emitting a signal, particularly electromagnetic waves, into the pharmaceutical product; and
- measuring a signal response, particularly a resonance frequency, based on an influence of the pharmaceutical product on the signal.

2. Method according to claim 1, wherein the measuring step comprises to measure a shift of a resonance frequency caused by the presence of the pharmaceutical product.

3. Method according to claim 1 or 2, wherein the method comprises the step of:
- evaluating the moisture content of the pharmaceutical product based on the measured signal response.

4. Method according to any one of claims 1-3, wherein at least one resonator (12a, 12b, 12c), particularly a split-cavity resonator, is used to determine the moisture content.

5. Method according to any one of claims 1-4, wherein the pharmaceutical product is arranged in a measuring zone of the resonator by means of a carrier (14) holding the pharmaceutical product.

6. Method according to any one of claims 1-5, wherein the method comprises the steps of:
- measuring a response of the signal, particularly a shift of the resonance frequency, of at least one sample having a known moisture content; and
- generate calibration data, preferably a calibration curve, based on the measured response.

7. Method according to claim 6, wherein the method comprises the further step of performing a Karl-Fischer analysis to determine the moisture content of the at least one sample.

8. Method for producing a pharmaceutical product, particularly a pharmaceutical patch (40), comprising:
- fabricating, particularly casting and/or assembling, the pharmaceutical product; and
- carrying out the method for determining the moisture content of a pharmaceutical product according to any one of claims 1-7.

9. Method according to claim 8, wherein the method comprises the step of:
- drying the pharmaceutical product based on the determined moisture content.

10. Method according to claim 9, wherein drying is terminated if a predefined moisture content is determined by carrying out the method for determining the moisture content of a pharmaceutical product.

11. Method according to any one of claims 8-10, wherein the step of determining the moisture content of the pharmaceutical product is carried out at various positions in the fabrication process and/or at various time points during the fabrication process.

12. Method according to any one of claims 8-11, wherein the method is a method of producing a, preferably dissolvable, microneedle array patch (40), wherein the fabricating step comprises:
- casting a microneedle tip, preferably in a microneedle mold (16), and
- casting a backing layer (28) connected to the microneedle tip, preferably in the microneedle mold (16).

13. Method according to any one of claims 8-12, wherein the step of determining the moisture content is carried out before casting the microneedle tip, and/or between casting the microneedle tip and casting the backing layer (28), and/or after casting the backing layer (28).

14. System (10) for determining the moisture content of a pharmaceutical product, particularly of a pharmaceutical patch, comprising:
- at least one resonator and
- a carrier (14) for the pharmaceutical product,
wherein the carrier (14) is adapted to arrange the pharmaceutical product in the measuring zone of the resonator.

15. System (10) for manufacturing a pharmaceutical product, particularly a pharmaceutical patch, comprising:
- a system for determining the moisture content according to claim 14, and
- a manufacturing device, particularly a casting device (22), for the production of the pharmaceutical product.
